Europäisches Patentamt

European Patent Office

Office européen des brevets

(19)

(11) **EP 1 196 784 B1**

(12) ## EUROPEAN PATENT SPECIFICATION

(45) Date of publication and mention
of the grant of the patent:
**17.09.2003 Bulletin 2003/38**

(21) Application number: **00942318.7**

(22) Date of filing: **14.07.2000**

(51) Int Cl.$^7$: **G01N 33/94**, G01N 33/493

(86) International application number:
**PCT/IB00/00966**

(87) International publication number:
**WO 01/006251 (25.01.2001 Gazette 2001/04)**

(54) **PROCESS FOR THE QUANTITATIVE DETERMINATION OF ALKALOIDS SUCH AS COCAINE IN A SOLID SAMPLE AND REAGENT FOR USE IN SUCH PROCESS**

VERFAHREN ZUR QUANTITATIVEN FESTSTELLUNG VON ALKALOIDEN WIE KOKAIN IN EINER FESTEN PROBE UND REAGENZ ZUR BENUTZUNG IN DIESEM VERFAHREN

PROCEDE PERMETTANT LA DETERMINATION QUANTITATIVE D'ALCALOIDES, TELS QUE LA COCAINE, DANS UN ECHANTILLON SOLIDE, ET REACTIF A UTILISER DANS CE PROCEDE

(84) Designated Contracting States:
**AT BE CH CY DE DK ES FI FR GB GR IE IT LI LU MC NL PT SE**

(30) Priority: **20.07.1999 IT VR990059**

(43) Date of publication of application:
**17.04.2002 Bulletin 2002/16**

(73) Proprietor: **Azienda Provinciale Per I Servizi Sanitari**
**38100 Trento (IT)**

(72) Inventors:
- **VITALONE, Vincenzo**
**I-38040 Martignano (IT)**
- **LOTTI, Andrea**
**I-38063 Avio (IT)**
- **GOTTARDI, Massimo**
**I-38034 Cembra (IT)**

(74) Representative: **Vannini, Mario et al**
**Maroscia & Associati S.r.L.,**
**Contra's. Caterina, 29**
**36100 Vicenza (IT)**

(56) References cited:
**WO-A-93/03368**      **US-A- 5 910 419**

- **SEGURA J ET AL: "Immunological screening of drugs of abuse and gas chromatographic-mass spectrometric confirmation of opiates and cocaine in hair" JOURNAL OF CHROMATOGRAPHY B: BIOMEDICAL APPLICATIONS,NL,ELSEVIER SCIENCE PUBLISHERS, vol. 724, no. 1, 5 March 1999 (1999-03-05), pages 9-21, XP004159001 ISSN: 0378-4347**
- **TAGLIARO F ET AL: "Hair analysis, a novel tool in forensic and biomedical sciences: new chromatographic and electrophoretic/electrokinetic analytical strategies" JOURNAL OF CHROMATOGRAPHY B: BIOMEDICAL SCIENCES & APPLICATIONS,NL,ELSEVIER SCIENCE PUBLISHERS, vol. 689, no. 1, 7 February 1997 (1997-02-07), pages 261-271, XP004054195 ISSN: 0378-4347**

EP 1 196 784 B1

## Description

This invention relates to a process for the quantitative determination of cocaine and other alkaloids, such as morphine and methadone, in a solid sample, e.g., a sample of hair, by using a screening type approach.

The invention also relates to a reagent for use in such process and new diagnostic kits including such reagent among their components.

By "screening type approach" is meant a kind of analysis permitting to analyze in a relatively short time span a relatively large number of samples in a cheap, efficacious and standardized manner. This kind of analysis permits to exclude the negative samples by immediately identifying the samples that do not contain the substance or the entire substance class or those in which said substances are present at a level lower than the threshold or cut-off value.

The threshold or cut-off is a practical limit selected to establish if the sample analyzed is positive or negative. The threshold value differs from the limit of detection of the method, that is, the lowest concentration of an analyte that can be determined. In fact, the cut-off value is normally set at a concentration higher than the limit of detection in order to obviate the imprecision of the analysis at values close to the limit of detection.

Cut-off values are conventionally established and take into account a multiplicity of factors, such as the capability of using commercially available reagents, the pharmacokinetic properties of the substances and the need to avoid false negatives (see: P.Zuccaro, S.Pichini, I.Altieri and R.Pacifici: Proposta di linee guida per l'analisi delle sostanze d'abuso nei liquidi biologici The substances that the metabolism of the human body accumulates in the hair are numerous. Usually, it is required to detect the presence of alkaloids and other substances of abuse, such as, morphine, methadone and/or cocaine. By the method of this invention further substances can also be detected, such as, those of the following non-limitative list: 6-O- mono-acetyl-morphine, bi-acetyl-morphine, codeine, papaverine, nalorphine, nicotine, cotinine, caffeine, noscarpine, mepivacaine, trimetropin, buprenorphine, pentazocyne, methadone metabolyte, benzoylecgonine, amphetamine, metamphetamine, methylenedioxyamphetamine, methylenedioxymetamphetamine, methylenedioxyethylamphetamine, benzodiazepines, barbiturates.

<u>Description of the state of the art</u>

Several techniques are known for the determination of the analytes of interest, such as those mentioned above. Specifically, for the analysis of cocaine that is present in hair, gas chromatography (GC) combined with Mass Spectrometry (MS) hereinafter referred to as GC/MS and Radio Immune Assay technique (R.I.A.) are known.

GC or liquid chromatography (LC) combined with MS are methods of resolution, purification or separation and identification of components of complex mixtures of organic or inorganic substances having even strictly similar chemical properties. The separation of substances dissolved in a liquid or fixed on a finely divided or porous solid substance is based on percolation, respectively elution trough them of an eluent gas, respectively liquid.

When the substances to separate/detect can be made gaseous and as eluent a gas is used, GC applies. The latter is a separation method based on the distribution between a solid or liquid stationary phase and a mobile phase made of the gases or vapors to separate, which are carried by a stream of an inert gas.

The analytical results are reported in a graph named chromatogram, in which the quantities of the single components present in the mixture and transferred to the eluent gas/liquid are reported versus time. The graph has peaks whose highness is a direct function of the quantity of the specific substance.

Chromatographic methods that can be used for confirmatory analyses are GC and LC, the latter being often referred to as High Performance Liquid Chromatography (HPLC). The most commonly employed detectors for GC are electron scatting detectors and phosphor nitrogen detectors.

As said above, GC/MS employs a gas chromatograph coupled to a mass spectrograph. By so doing, the separation capability of GC combines with the specificity proper of MS. Therefore, GC/MS represents the method of choice for confirmatory analyses of the above named substances as well as their metabolites (see again "Proposta di linee guida per l'analisi delle sostanze d'abuso nei liquidi biologici" by ISTITUTO SUPERIORE DI SANITA')

In the conventional acid hydrolysis, however, cocaine is extracted by hydrochloric acid as it is, i.e. without undergoing transformation into its metabolite benzoylecgonine. Since the amounts of cocaine extracted in this way are very little, it is not possible to determine cocaine by the usual screening methods.

Radio Immune Assay (RIA) is based on radio-immunological tests using known amounts of antibodies and of analyte labeled with a radioisotope (generally $I^{125}$). During incubation the labeled analyte and that possibly present in the sample compete for the antibody sites. After precipitation of antigen-antibody complexes and centrifugation the supernatant or the precipitate are transferred to a gamma Geiger counter that measures the radioactivity level. RIA kits are extremely sensitive and allow identification of 1-5 ng of substance per ml. Adoption of automatic instruments for pipetting and counting allows the contemporaneous analysis of numerous samples with response times of 1 to 5 hours. On the other hand, the use of radioactive isotopes requires adequate safety measures. Furthermore, the relatively short half-life of the radioactive isotopes imposes a careful handling of reagents.

RIA does not lend itself to a widespread use for the quick determination of the substances in question in view, first of all, of the high cost of reagents, further increased by liability of the antisera. Secondly, in view of the criticality of the analysis due to the need of operating with radioactive materials that are dangerous for the analysts and also in view of the required times which are relatively long. RIA also requires the availability of rooms properly equipped and shielded, as well as particular care in handling waste materials and in their disposal.

Therefore, RIA is scarcely employed in this sort of analyses. The prior art shows therefore the impracticality of determining cocaine present in the hair by the so-called screening type approach, as described above, i.e., by very quick and cheap techniques as are used for the determination of the same substances in urine. This is mainly due to the fact that in the conventional screening-type approach what is analyzed is not cocaine, but its metabolite benzoylecgonine. The fact that this substance does not exist in nature by itself, but only as the metabolite of cocaine and the fact that the reaction of transformation of cocaine into benzoylecgonine, i.e. the transformation of the ester group into an hydroxyl group, is irreversible in an alkaline environment render the determination of benzoylecgonine a necessary and sufficient condition for the detection of cocaine.

Benzoylecgonine is found in the hair in a ratio of 1 to 4 or even 1 to 10 and more with respect to cocaine. Therefore, when the amount of cocaine is very low or close to the cut-off values (0.2-0.1 ng cocaine per mg hair) screening cannot be performed because of the low amount of this metabolite (0.05-0.025 ng benzoylecgonine per mg hair), such amount being undetectable by the screening apparatuses available in the laboratories.

US-A-5 910 419 which is the nearest prior art to the invention relates to a method for screening hair samples using the well known ELISA technique for the presence of cannabinoids and further RIA for the presence of cocaine. As already outlined, the radio immunoassay does not lend itself to quick and cheap determination of drug of abuse and further involves safety and handling difficulties.

Summary of the invention

The main object of this invention is to overcome the above mentioned drawbacks, i.e. the impossibility to dose the cocaine present in a solid sample by the conventional screening-type approach.

In accordance with the invention, this object is achieved by a screening-type procedure for the quantitative determination of cocaine and other alkaloids which are present in a solid sample which, in accordance with claim 1, includes the following steps:

a) preparing a solid sample in a finely divided or powdered form;

b) selecting a liquid reagent providing constant concentration of hydroxyl groups suitable for extracting and transforming cocaine into benzoylecgonine and for extracting other similar substances;

c) extracting cocaine and other similar substances contained in the sample and transforming the extracted cocaine into benzoylecgonine by maintaining the sample completely immersed in said liquid reagent at a temperature ranging from 10°C to 250°C for a period of time ranging from few seconds to 48 hours; and

d) analysing the liquid separated from the solid sample to determine the concentration of benzoylecgonine contained in said liquid with respect to the cut-off limit using a conventional screening kit for the determination of the said substance in urine.

Preferred aspects of the invention include using hair as the solid material forming the solid sample; using a buffer as the reagent which provides hydroxyl groups, more preferably an ammonia buffer, most preferably a buffer that is hereinafter referred to as VMA; and heating the sample immersed in the liquid at a temperature of about 100 to 150°C for about one hour.

In accordance with another aspect of the invention a process is provided which includes the additional steps of:

- arranging samples by increasing concentrations of the substances of interest; and
- performing confirmation analyses with known techniques, such as, GC or GC/MS.

In one embodiment of the invention, the process may provide the following steps:

- providing a sample made of about 50 to 300 mg of finely divided and/or powdered material;
- adding in the test tube containing the said sample a suitable liquid reagent until the sample is completely immersed, said reagent being capable of performing extraction and transformation of cocaine into benzoylecgonine and at the same time of extracting other similar substances which are present in the sample
- if necessary, agitating the test tube to facilitate immersion of the sample;
- heating the contents of the test tube to a temperature T1 for a time interval t1 by keeping the test tube immersed in a thermostated bath or by placing it in an oven;

- cooling the test tube to room temperature;
- taking the liquid and transferring it into a test tube suitable for a screening type instrument;
- performing the screening by using a kit of reagents for the determination of the said substances in urine;
- reading the data resulting from the first level instrumentation to verify the concentration values with respect to the cut-off limit; and
- contemporaneously determining the amount(s) of substance(s) present.

In accordance with another aspect of the invention a reagent is provided for use in the above mentioned process which-in the most -preferred embodiment has the following formula:

$$VMA = 0.2\ M\ (NH^4)^2HPO^4 + 5ml/L\ 25\%\ NH^4OH$$

In the above formula $(NH_4)_2HPO_4$ is dibasic ammonium phosphate and $NH_4OH$ is ammonium hydrate. In fact, 5ml/L $NH_4OH$ give a 0.07 M concentration of hydroxyl groups, which is comprised in the range giving 100% conversion.

In addition to the main advantage of the invention as outlined above, another advantage is that the confirmation analyses (e.g., GC/MS) need to be performed only on those samples that have been determined to be positive at the initial screening whereas, when conventional techniques are used, all samples must be analyzed since there is no kit available for determination of cocaine, but only for determination of benzoylecgonine.

In average, the analysis of 25 samples requires two hours for the preparation of the samples plus one week for the confirmation analyses of all (both positive and negative) samples.

On the other hand, by applying the present invention, an analyst will require for the same 25 samples two hours for samples preparation plus 30 minutes for the screening-type analysis plus the time necessary for performing the confirmation analyses. However, the latter need to be performed only on those samples which have been determined to be positive.

Since, in the average, positive samples are between 0 and 20% and since most of the time required is spent in the confirmation analyses, the procedure according to the present invention allows saving of substantial time which is estimated at about 70 to 90% of the time required for the analysis of the 25 samples taken into consideration.

Another advantage according to this invention is that the possible dragging of the active substances from one sample to another in the confirmation analyses is eliminated or minimized because it is possible to arrange samples in order of increasing concentration of the substances of interest, therefore proceeding to confirmation analyses starting from those samples having the lowest concentration and then with those having higher and higher concentrations.

In this way, it is completely eliminated the possibility that a sample with high concentration of, e.g. cocaine, leaves a trace of it in the instrument used for the analysis and has an impact on the determination of the same substance in the following sample, perhaps having a concentration just below the cut-off.

Still another advantage of this invention is that it allows to search and determine at the same time and in the same sample not only cocaine but also other substances, such as, morphine by applying the same cut-off limit or else methadone by modifying the limit of cut-off.

One further advantage is that the procedure according to this invention offers a higher guarantee to the analyst because positive outcome is confirmed by two different analytical methods.

In a further aspect of the invention a diagnostic kit is provided including the reagent described above as one of its components.

Still further additional advantages will appear from the reading of the following detailed description and the following non-limitative examples.

Brief Description of the Drawings

Figure 1 is a chromatogram obtained from an apparatus performing gas chromatography on the sample of Table 2;

Figure 2 is the graph obtained by Mass Spectrometry on the first sample after acid hydrolysis and shows presence of cocaine;

Figure 3 is the Mass Spectrum of the same first sample after treatment according to the present invention and shows presence of benzoylecgonine;

Figure 4 is the Mass Spectrum of the same first sample after treatment according to the present invention and shows presence of morphine;

Figure 5 is a chromatogram similar to that of Figure 1 and refers to the first sample treated according to this invention as shown in Table 3;

Figure 6 is similar to the MS of Figure 3 showing presence of benzoylecgonine;

Figure 7 is similar to the MS of Figure 3 showing presence of morphine;

Figure 8 is similar to Figure 1 but relates to a second sample;

Figure 9 is similar to Figure 5 but relates to a second sample;

Figures 10 and 11 are respectively similar to Figures 8 and 9 but relate to a third sample;

Figures 12 and 13 are respectively similar to Figures 8 and 9 but relate to a fourth sample;

Figure 14 is a diagram showing transformation of cocaine into its metabolite benzoylecgonine;

Figure 15 is a graph showing concentration of cocaine versus concentration of $OH^-$ when the reaction of transformation of cocaine into benzoylecgonine occurs at 100° C for 1 hour; and

Figure 16 is similar to Figure 15 and shows the case in which the reaction occurs at 150°C for 1 hour.

Detailed description of invention embodiments

The process of this invention substantially is a process for the quantitative extraction and transformation of cocaine into benzoylecgonine and for the extraction of similar alkaloids, in particular toxic substances of abuse and/ or drugs, which are present in a sample, prepared starting from a solid material.
In the following detailed example, the sample is obtained starting from finely divided hair.
The procedure allows, in particular, the dosage of cocaine by a screening type technique, at the same time allowing the dosage of possible other toxic substances and the like present in the hair, by using kits of reagents available in commerce and intended for the analysis in urine. The following steps are carried out:

- crush hair in fragments of two-three mm length;
- weigh the fragments to form a sample having a weight of 50 to 300 mg;
- wash the sample with methanol in a closed test tube at room temperature to eliminate possible external substances that might interfere with the results, such as, for example, traces of drugs external to the hair that have deposited on it because of its presence in the air (by so doing, it is possible to distinguish if the hair was that of a handler or that of the consumer of the drug);
- Repeat the step of washing with methanol, if necessary;
- wash the sample with ethanol in a closed test tube to eliminate traces of methanol or water;
- dry in an oven at about 45°C under flow of inert gas, such as, nitrogen;
- add into the test tube 0.5 to 2 ml of the reagent as defined above and shake if necessary;
- heat the test tube to 100°C and maintain at this temperature for 1 hour, e.g. by means of a thermostatic bath or an oven;
- cool the test tube to room temperature, e.g., by immersing the tube into cold water or simply leaving it at room temperature for a suitable time span;
- take the liquid and pour into a test tube of the kind used for urine examination (if necessary, centrifuge the test tube to eliminate turbidity);
- insert the tube in a "first-level screening apparatus" for the quantitative determination of the substances sought for (benzoylecgonine, morphine, methadone, etc);
- adjust settings of the first-level apparatus in a way suitable for small amounts (this may be necessary if the apparatus is alternatively used for determination in hair or urine);
- perform screening -type analyses using the reagents provided with the kit normally employed for the examination of urine;
- read data resulting from the first-level analyses and establish positivity or negativity with reference to the cut-off value.

The above procedure may then be complemented with the following additional steps when confirmation analyses are required:

- arrange the samples in the order of increasing concentration of the sought for substances, determined as above described;
- perform confirmation analyses by analyzing the samples taken in the order of the arrangement (In this way the above mentioned problems of dragging traces of drugs from one sample to the other are overcome).

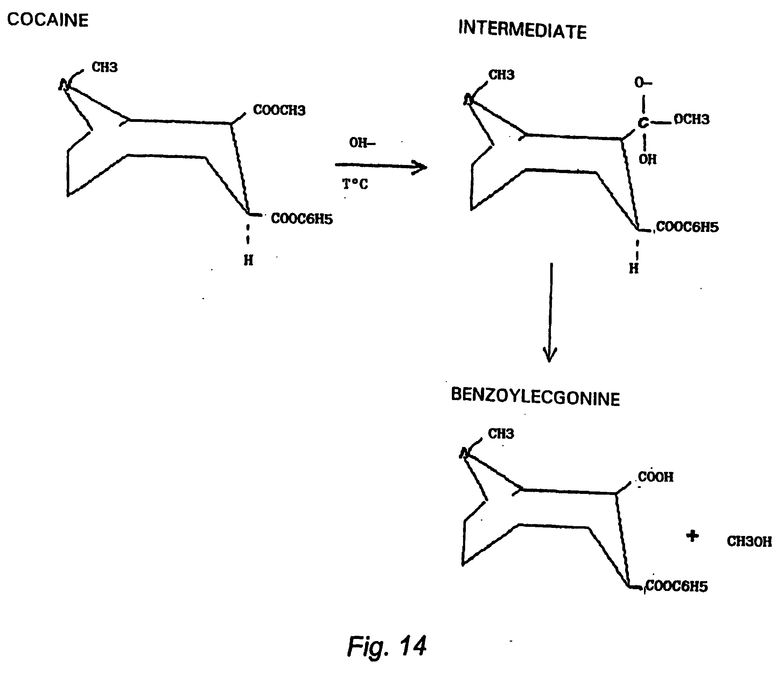

The invention process provides for the use of a reagent (hereinafter referred to as VMA) which is a buffer solution. The buffer serves for transforming the cocaine present in the hair into its metabolite benzoylecgonine, as shown in the scheme of Figure 14. Cocaine, in the presence of hydroxyl groups and at a suitable temperature first is transformed into an intermediate product and then into benzoylecgonine plus methanol.

Buffer solutions are obtained by reacting a salt with its weak base. These solutions have a stable pH; therefore the VMA reagent is able to produce hydroxyl groups in a steady way. The use of solutions in which the production of hydroxyl groups is not regular creates problems when the cocaine concentration is close to the cut-off limit.

As said above, the composition of the buffer for use in the process is preferably the following:

$$VMA = 0.2 \text{ M } (NH_4)_2 HPO_4 + 5ml/L \text{ } 25\% \text{ } NH_4OH$$

Alternatively, VMA may be replaced by solution in which the component being the source of hydroxyl groups is selected among the following non-limitative list of substances: aluminum hydroxide, barium hydroxide octahydrate, benzyltriethylammonium hydroxide, benzyltrimethylammonium hydroxide, calcium hydroxide, lithium hydroxide, lithium hydroxide monohydrate, magnesium hydroxide, potassium hydroxide, potassium hydroxyantimoniate, sodium hydroxide, sodium hydroxide monohydrate, strontium hydroxide octahydrate, tetramethylammonium hydroxide, tetrapropylammonium hydroxide, trimethylvinylammonium hydroxide.

As solvent, any of the following can be used in alternative:

ethanol, methanol, water, monobasic ammonium phosphate, ammonium acetate, ammonium benzoate, ammonium bicarbonate, ammonium bichromate, ammonium bisulphate, ammonium bromide, ammonium carbamate, ammonium carbonate, ammonium citrate bibasic, ammonium chromate, ammonium iodide, molibdate, ammonium monovanadate, ammonium nitrate, ammonium oxalate monohydrate, ammonium persulphate, ammonium sulphate, ammonium sulphamate, ammonium sulphite, ammonium sulphide, ammonium tartrate, ammonium thiocyanate, ammonium thioglycolate, ammonium thiosulphate, ammonium chloride, sodium phosphate monobasic, sodium phosphate bibasic, potassium phosphate monobasic, potassium phosphate bibasic.

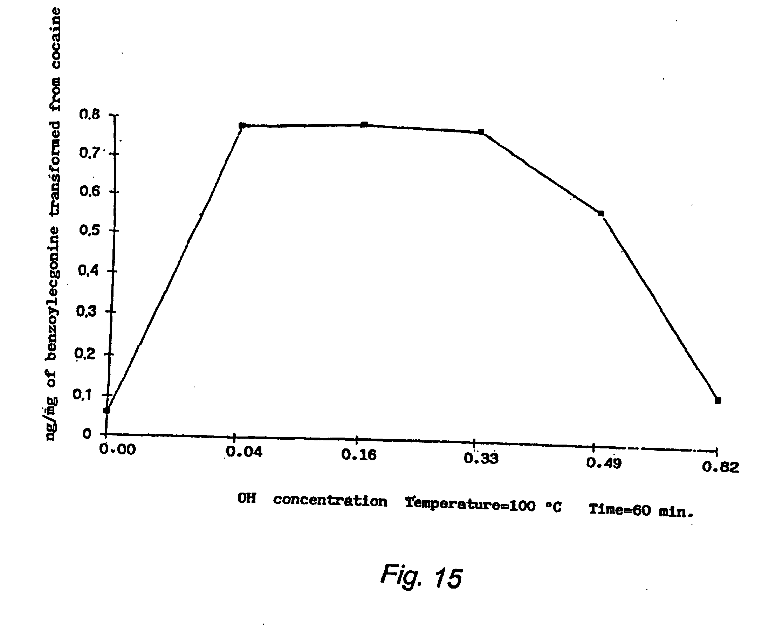

Figure 15 shows a graph in which the percentage of cocaine transformed into benzoylecgonine is reported versus the concentration of OH$^-$ when the reaction is carried out at the temperature of 100°C for 1 hour. From the graph it appears evident that the transformation is maintained at high levels (at least 70%) for a range of OH$^-$ concentrations of from 0.03 to 0.5 M.

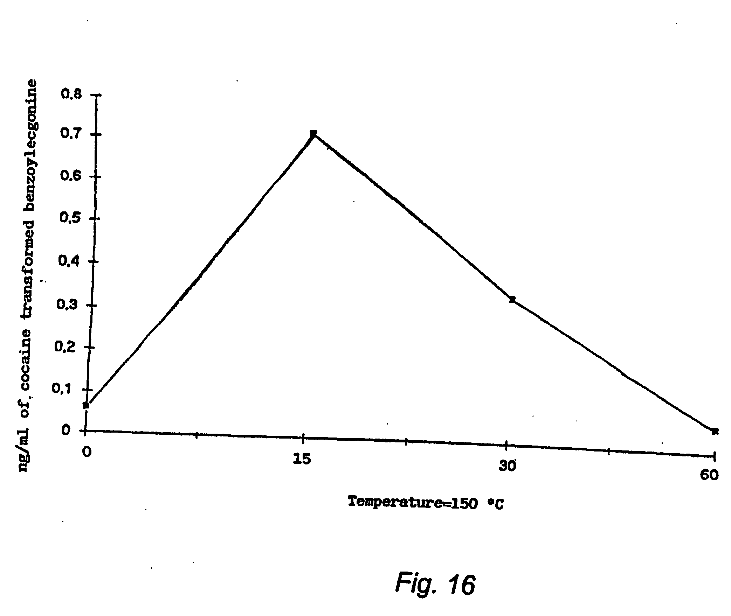

From the graph of Figure 16, which shows hydroxyl concentrations after, respectively, 0, 15, 30 and 60 minutes in a reaction carried out at 150°C; it appears evident that the percentage of transformed cocaine is high only around the abscissa point of 15 minutes.

From all of the above and numerous other experiments the optimal values for the reaction temperature and time result to be, respectively, 100°C and 1 hour.

The examples reported below show the quantitative determination of cocaine in hair performed both with known techniques and with the process of the invention. Analyses have been carried out using the following instruments: for the screening analyses, the ROCHE instrumentation named "COBAS MIRA PLUS" which uses reagents provided by the same company and named "ABUSCREEN ON LINE"; for GC/MS the instrument provided by the company VARIAN which is named "SATURN GC/MS", model 4D.

EXAMPLES

Before proceeding to the various screening analyses the apparatus has been controlled with a sample positive to both cocaine and morphine in order to verify feasibility of the methodology.

As can be seen from Table 1 the sample resulted positive to both cocaine and morphine:

TABLE 1

| SUBSTANCE | CONCENTRATION | POSITIVITY/NEGATIVITY |
|---|---|---|
| MORPHINE | 0.16 ng/ml | POSITIVE |
| COCAINE | 0.15 ng/ml | POSITIVE |

The concentration values detected by the apparatus resulted to be 0.16 for morphine and 0.15 for cocaine, in line with the expected values for both substances present in the control sample.

EXAMPLE 1

In this example a sample has been analyzed which resulted, in the end, to be positive to both cocaine and morphine.

The sample was subjected to conventional analysis with acid hydrolysis and then subjected to screening analysis which gave the following results:

TABLE 2

| SUBSTANCE | CONCENTRATION | POSITIVITY/NEGATIVITY |
|---|---|---|
| MORPHINE | 0.13 ng/ml | POSITIVE |
| COCAINE | 0.01 ng/ml | NEGATIVE |

As can be seen, acid hydrolysis was able to extract an amount of morphine higher than the limit, but the amount of cocaine resulted to be too little to allow detection by the screening apparatus. The reason is that cocaine was extracted as such and not transformed into its metabolite benzoylecgonine, which latter is just what present screening methods detect.

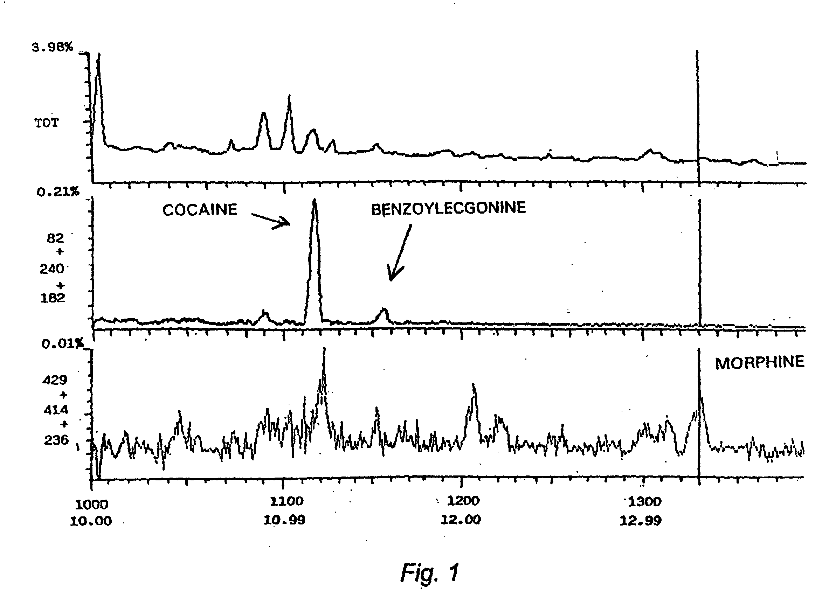

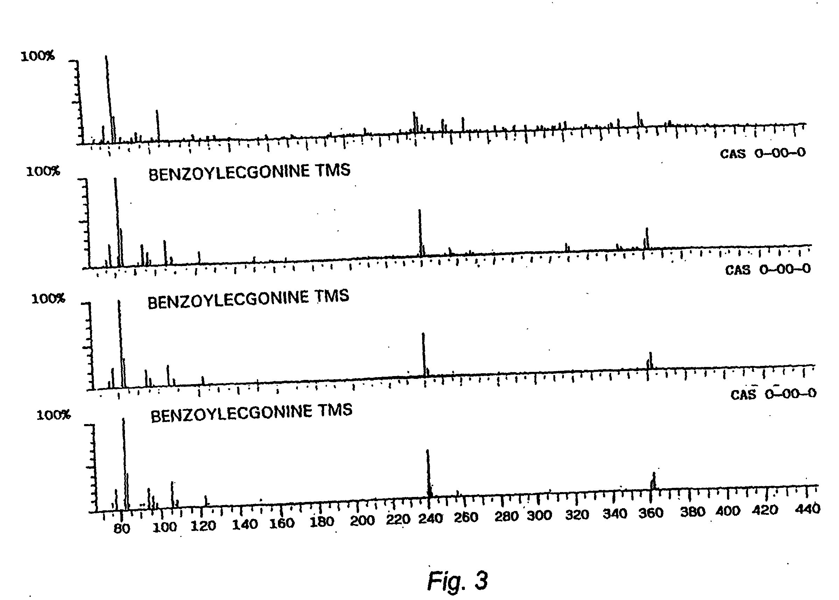

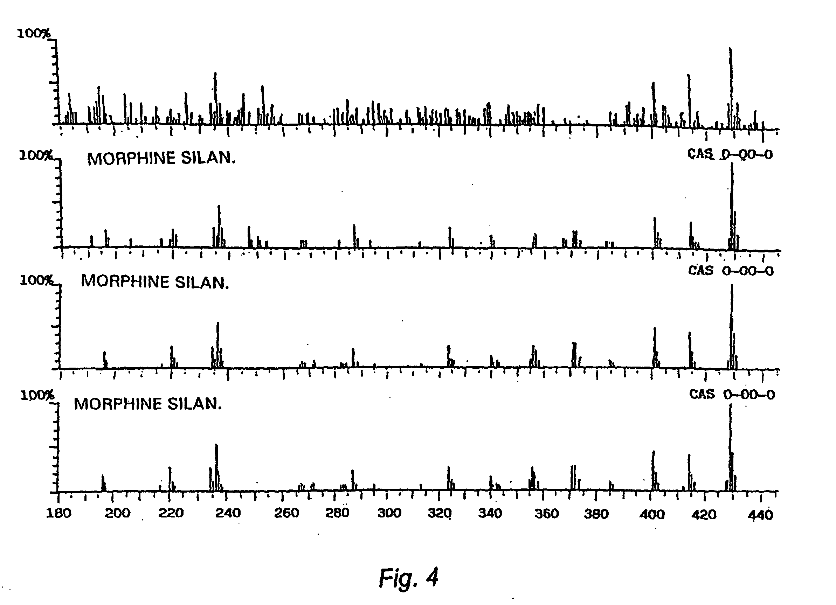

The same sample was then subjected to confirmatory analysis by GC/MS with the results reported in Figures 1 to 4. Specifically, from Figure 1 it can be seen that the chromatogram of the first sample shows a high peak at the position of cocaine and a very small peak at the position of benzoylecgonine.

The same Figure 1 also shows confirmation of the presence of morphine.

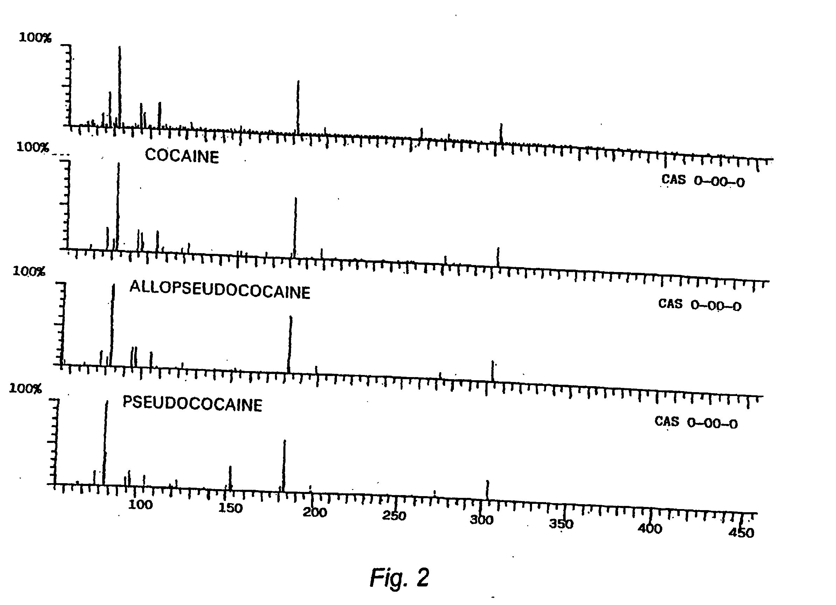

Additional confirmations of the presence of both cocaine and morphine result from the graphs of Figures 2, 3 and 4.

The sample of Example 1 was then subjected to the procedure of this invention to give the results shown in Table 3.

TABLE 3

| SUBSTANCE | CONCENTRATION | POSITIVITY/NEGATIVITY |
|---|---|---|
| MORPHINE | 0.23 ng/ml | POSITIVE |
| COCAINE | 0.37 ng/ml | POSITIVE |

It is immediately clear that the values detected for both morphine and cocaine are higher than the respective limits, therefore the sample is positive for both.

The high value detected for cocaine is to be ascribed to the benzoylecgonine that has been produced during the transformation reaction.

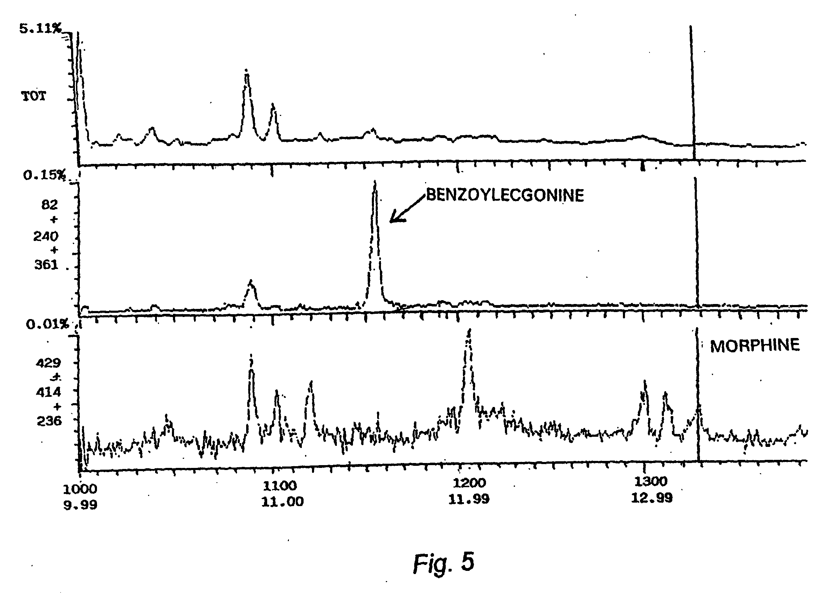

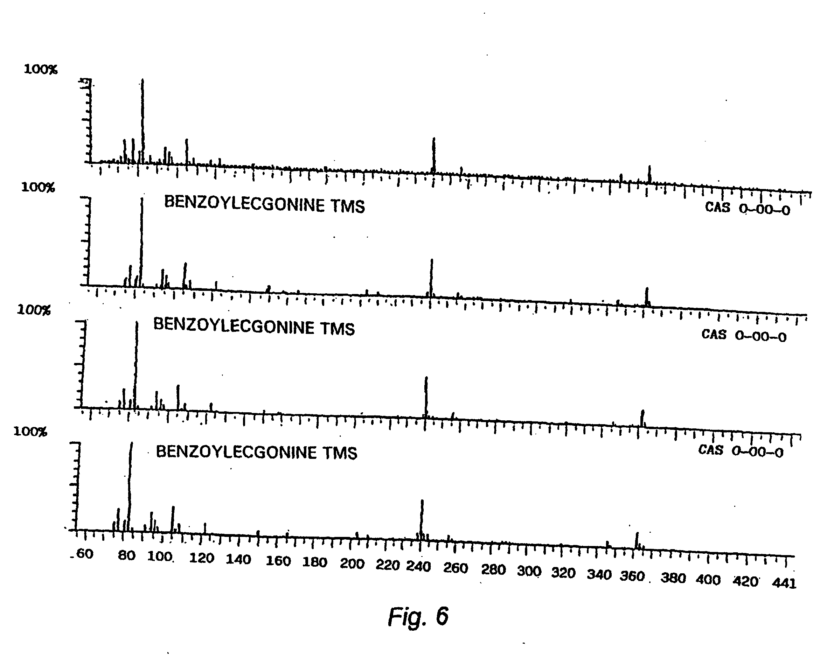

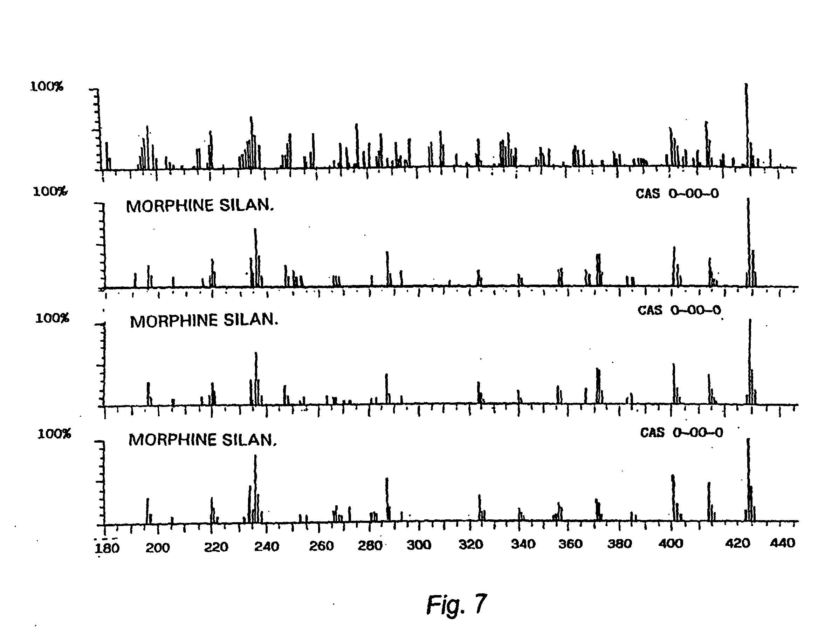

The same sample of Example 1 treated with the process of this invention has been subjected to GC/MS confirmation analyses with the results reported in Figures 5 to 7. As can be seen from Figure 5, the cocaine peak practically cannot be seen anymore because cocaine has been completely transformed in benzoylecgonine, the peak of which is well apparent in the same Figure 5.

EXAMPLE 2

Again in this example a sample which, in the end, resulted to be positive to both cocaine and morphine has been analyzed first by the conventional analytical method of acid hydrolysis and screening with the results reported in Table 4.

TABLE 4

| SUBSTANCE | CONCENTRATION | POSITIVITY/NEGATIVITY |
|---|---|---|
| MORPHINE | 0.32 ng/ml | POSITIVE |
| COCAINE | 0.03 ng/ml | NEGATIVE |

Like in the preceding Example, acid hydrolysis was able to extract an amount of morphine higher than the limit, but the quantity of cocaine extracted was too little to allow detection by the apparatus.

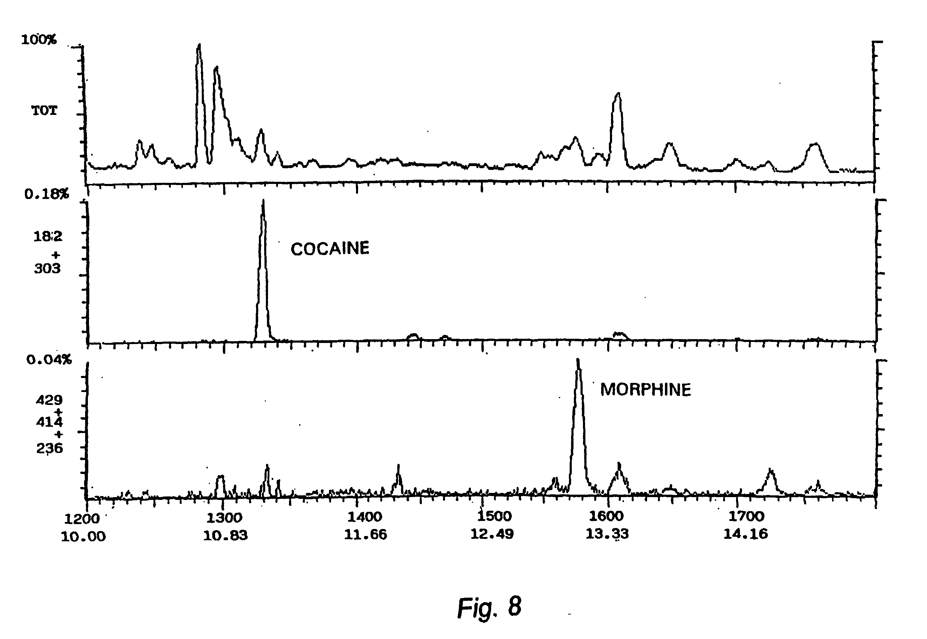

The sample was then subjected to GC/MS confirmatory analyses to give the results reported in Figure 8 where it can be seen that the chromatogram shows a high peak at the position of cocaine and a negligible peak at the position of benzoylecgonine.

The sample of Example 2 was then subjected to the invention process and to screening analyses with the results reported in Table 5.

TABLE 5

| SUBSTANCE | CONCENTRATION | POSITIVITY/NEGATIVITY |
|---|---|---|
| MORPHINE | 0.28 ng/ml | POSITIVE |
| COCAINE | 0.99 ng/ml | POSITIVE |

Both the cocaine and the morphine values are higher than the respective limits, therefore the sample is positive to both.

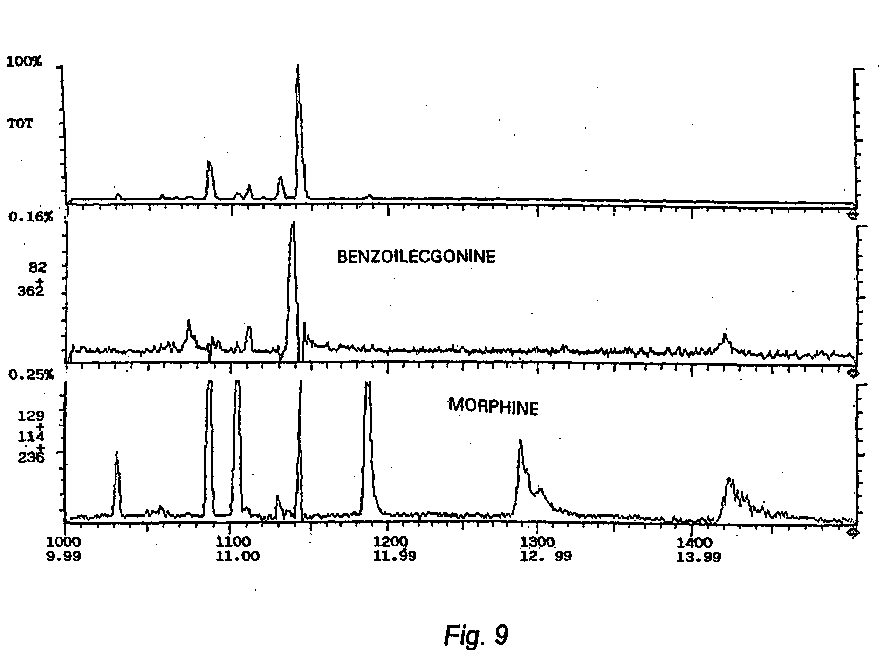

The sample of this Example, treated according to the invention process, was also subjected to GC/MS confirmation analyses with the results reported in Figure 9 where it can be seen that the cocaine peak is not practically present anymore whereas the benzoylecgonine peak is high.

EXAMPLE 3

In this Example a sample has been analyzed which, at the end, resulted to be positive to cocaine only and not to morphine.

This sample was first subjected to acid hydrolysis and screening by the conventional methods to give the results reported in Table 6.

TABLE 6

| SUBSTANCE | CONCENTRATION | POSITIVITY/NEGATIVITY |
|---|---|---|
| MORPHINE | 0.02 ng/ml | NEGATIVE |
| COCAINE | 0.01 ng/ml | NEGATIVE |

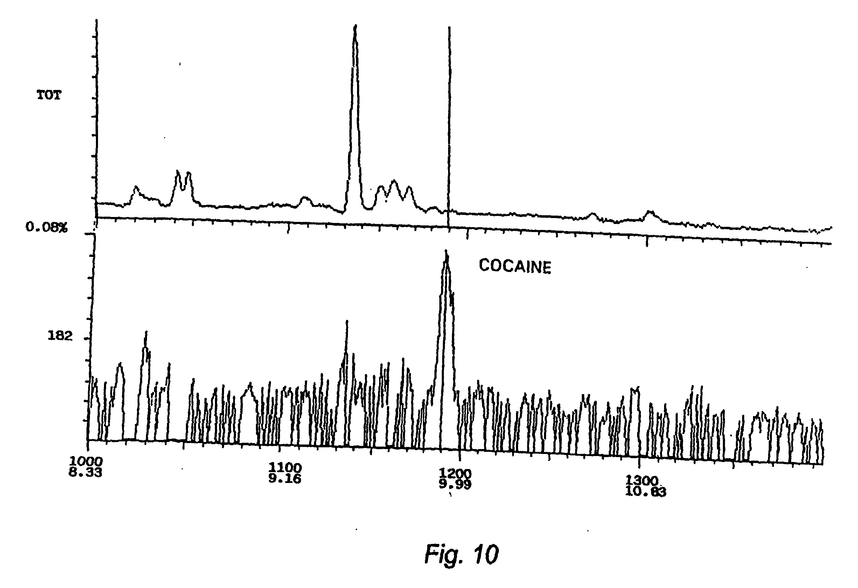

By acid hydrolysis a quantity of morphine lower than the limit was extracted. The quantity of cocaine was also too little to allow the equipment to detect it. By using the methods known in the art, therefore, nothing can be said as to the positivity or negativity of the sample and it was necessary to make recourse to the GC/MS confirmation analyses that gave the results reported in Figure 10. In such Figure, the chromatogram of the sample of this Example shows a high peak at the position of cocaine and a negligible peak at the position of benzoylecgonine. This confirms once more that acid hydrolysis extracted cocaine as such without any transformation.

The same sample of this Example has also been treated according to the invention process and subjected to screening analysis. Results are reported in Table 7.

TABLE 7

| SUBSTANCE | CONCENTRATION | POSITIVITY/NEGATIVITY |
|---|---|---|
| MORPHINE | 0.03 ng/ml | NEGATIVE |
| COCAINE | 0.13 ng/ml | POSITIVE |

It is immediately evident that while the value of morphine is lower than the limit, that of cocaine is slightly higher than the cut-off and therefore the sample is only positive to cocaine.

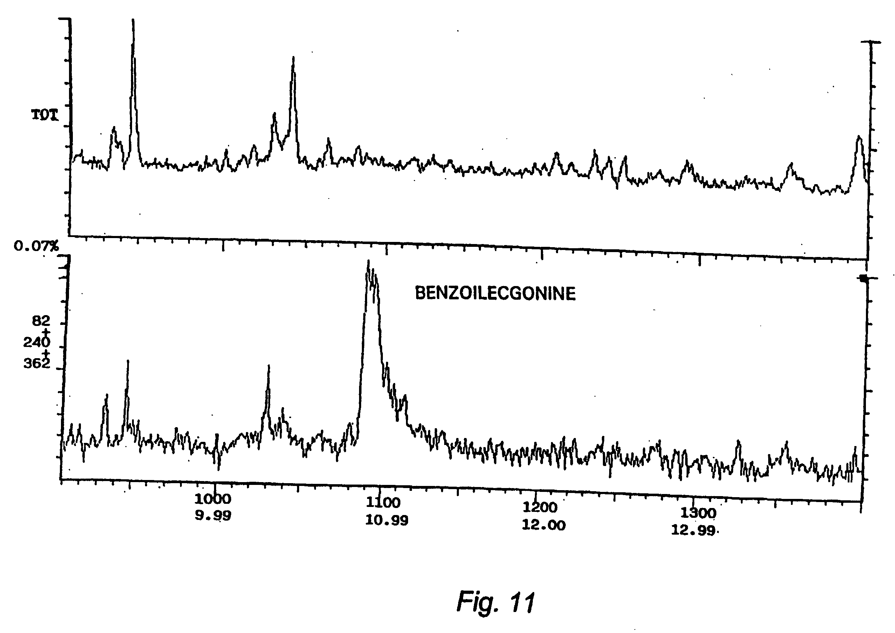

This same sample, after treatment with the invention process, has been subjected to GC/MS confirmation analyses with the results shown in Figure 11. In this Figure, the peak of cocaine has practically disappeared because the same was completely transformed into benzoylecgonine, the peak of which is, instead, very high and well apparent.

EXAMPLE 4

This fourth Example also analyzes a sample which, at the end, resulted positive to cocaine and not to morphine.

Analyses were carried out by applying the same scheme as in the preceding Examples.

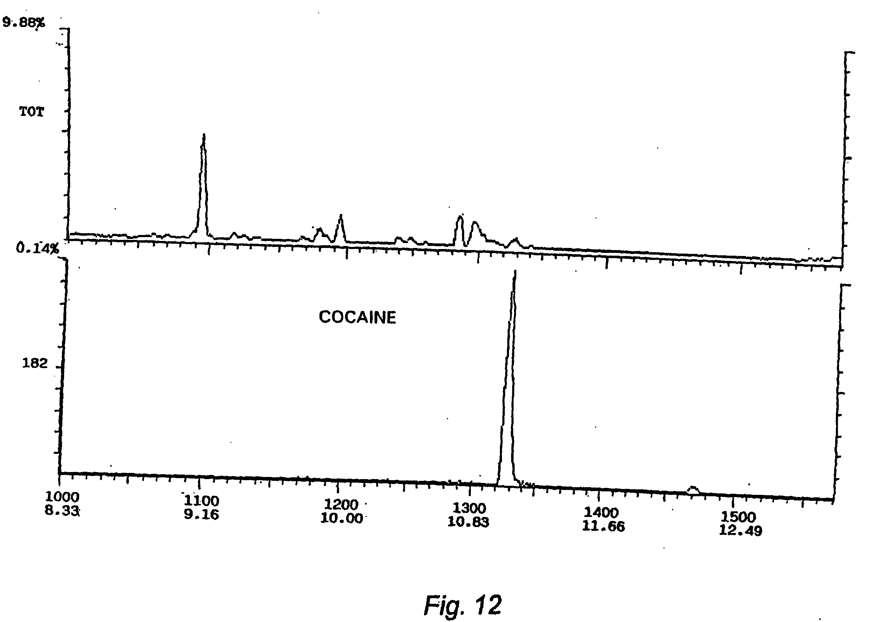

Table 8 shows that upon analysis with acid hydrolysis the sample seems to be completely negative to both morphine and cocaine, whereas Figure 12 indicates the presence of cocaine in an amount higher than the limit.

TABLE 8

| SUBSTANCE | CONCENTRATION | POSITIVITY/NEGATIVITY |
|---|---|---|
| MORPHINE | 0.02 ng/ml | NEGATIVE |
| COCAINE | 0.05 ng/ml | NEGATIVE |

The same sample, treated with the invention process (VMA reagent) already on the first screening analysis shows a presence of cocaine higher than the limit, see Table 9.

TABLE 9

| SUBSTANCE | CONCENTRATION | POSITIVITY/NEGATIVITY |
|---|---|---|
| MORPHINE | 0.02 ng/ml | NEGATIVE |
| COCAINE | 0.64 ng/ml | POSITIVE |

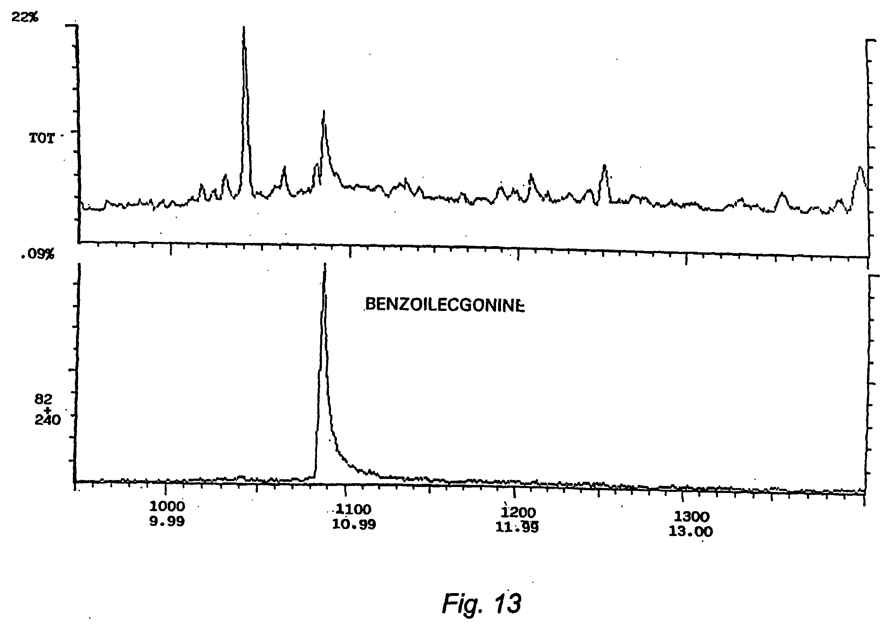

As usual, this result was confirmed by GC/MS analysis as shown in Figure 13. The big peak of benzoylecgonine indicates that, before the treatment, the sample contained cocaine in a percentage higher than the cut-off.

**Claims**

1.    A screening-type process for the quantitative determination of cocaine and other alkaloids which are present in a solid sample which includes the steps of:

   a) preparing a solid sample in a finely divided or powdered form;
   b) selecting a liquid reagent providing constant concentration of hydroxyl groups suitable for extracting and transforming cocaine into benzoylecgonine and for extracting other similar substances;
   c) extracting cocaine and other similar substances contained in the sample and transforming the extracted cocaine into benzoylecgonine by maintaining the sample completely immersed in said liquid reagent at a temperature ranging from 10°C to 250°C for a period of time ranging from few seconds to 48 hours; and
   d) analysing the liquid separated from the solid sample to determine the concentration of benzoylecgonine contained in said liquid with respect to the cut-off limit using a conventional screening kit for the determination of benzoylecgonine in urine.

2.    Process according to claims 1, wherein said solid sample is a sample of hair.

3.    Process according to claims 1 and 2, wherein said temperature is ranging from 100°C to 150°C.

4.    Process according to claims 1 and 2, wherein said period of time is ranging from 15 minutes to 24 hours.

5.    Process according to any preceding claims, wherein said temperature is maintained at 100°C for 1 hour.

6.    Process according to claim 1, wherein said liquid reagent is an ammonia buffer comprising 0.2 M (NH4)2HPO4 with the addition of 5 ml of 25% NH4OH to each liter thereof.

7.    Process according to claim 6, wherein the concentration of hydroxyl groups in said ammonia buffer is in the range of from 0.0001 M to 5 M.

**8.** Process according to claim 6, wherein the concentration of hydroxyl groups in said ammonia buffer is in the range of 0.03M to 0.5 M.

**9.** Process according to claim 6, wherein the concentration of hydroxyl groups in said ammonia buffer is in the range of 0.04M to 0.33 M.

**10.** Process according to any preceding claims, wherein the analyzed samples are arranged in increasing order of concentration of cocaine or other alkaloids.

**11.** Process according to claim any preceding claims, wherein the samples are subjected to confirmation analyses with standard techniques such as GC or GC/MS.

**12.** Process according to any preceding claims, wherein each hair sample is made of about 50mg to 300 mg of finely divided and/or powdered hair.

**13.** Process according to claim 1, wherein said liquid reagent is a solution comprising a solute selected among aluminum hydroxide, barium hydroxide octahydrate, benzyltriethylammonium hydroxide, benzyltrimethylammonium hydroxide, calcium hydroxide, lithium hydroxide, lithium hydroxide monohydrate, magnesium hydroxide, potassium hydroxide,potassium hydroxyantimoniate, sodium hydroxide, sodium hydroxide monohydrate, strontium hydroxide octahydrate, tetramethylammonium hydroxide, tetrapropylammonium hydroxide, trimethylvinylammonium hydroxide, dissolved in a solvent selected among ethanol, methanol, water, monobasic ammonium phosphate, ammonium acetate, ammonium benzoate, ammonium bicarbonate, ammonium bichromate, ammonium bisulphate, ammonium bromide, ammonium carbamate, ammonium carbonate, ammonium citrate bibasic, ammonium chromate, ammonium iodide, molibdate, ammonium monovanadate, ammonium nitrate, ammonium oxalate monohydrate, ammonium persulphate, ammonium sulphate, ammonium sulphamate, ammonium sulphite, ammonium sulphide, ammonium tartrate, ammonium thiocyanate, ammonium thioglycolate, ammonium thiosulphate, ammonium chloride, sodium phosphate monobasic, sodium phosphate bibasic, potassium phosphate monobasic, potassium phosphate bibasic.

**14.** Diagnostic kit for the carrying out of the process according to any claims 1 to 13, comprising a liquid reagent with constant concentration of hydroxyl groups suitable for extracting cocaine and other alkaloids and transforming cocaine into benzoylecgonine, and a conventional screening kit for the determination of benzoylecgonine in urine samples.

**Patentansprüche**

**1.** Analytisches Verfahren zur mengenmäßigen Bestimmung von Kokain und anderen Alkaloiden, die in einer festen Probe vorliegen, mit den folgenden Schritten:

    a) Vorbereiten einer festen Probe in fein unterteilter oder pulvriger Form,
    b) Auswählen eines flüssigen Reagens, das konstante Konzentration von Hydroxyl-Gruppen bereitstellt, die geeignet sind, Kokain zu extrahieren und in Benzoylecgonin um zu wandeln und zum extrahieren anderer ähnlicher Substanzen,
    c) Extrahieren von Kokain und anderer ähnlicher Substanzen, die in der Probe enthalten sind und Umwandeln des extrahierten Kokains in Benzoylecgonin, wobei die Probe vollständig untergetaucht in dem flüssigen Reagens gehalten wird bei einer Temperatur zwischen 10° C bis 250° C für eine Zeitdauer zwischen wenigen Sekunden bis 48 Stunden, und
    d) Analysieren der Flüssigkeit, die aus der festen Probe separiert wurde, um die Konzentration des in der Flüssigkeit enthaltenen Benzoylecgonin zu bestimmen relativ zu der Abscheidungs-Grenze, wobei ein herkömmliches Analysegerät verwendet wird für die Bestimmung von Benzoylecgonin im Urin.

**2.** Verfahren gemäß Anspruch 1, **dadurch gekennzeichnet, dass** die feste Probe eine Haarprobe ist.

**3.** Verfahren gemäß Ansprüchen 1 und 2, **dadurch gekennzeichnet, dass** die Temperatur zwischen 100° C bis 150° C ist.

**4.** Verfahren gemäß Ansprüchen 1 und 2, **dadurch gekennzeichnet, dass** die Zeitdauer zwischen 15 min bis 24 Stunden ist.

**5.** Verfahren gemäß einem der vorhergehenden Ansprüche, **dadurch gekennzeichnet, dass** die Temperatur bei 100° C gehalten wird für 1 Stunde.

**6.** Verfahren gemäß Anspruch 1, **dadurch gekennzeichnet, dass** das flüssige Reagens ein Ammoniakpuffer ist, der 0,2 M (NH4)2HPO4 mit dem Zusatz von 5 ml von 25% NH4OH zu jedem Liter davon enthält.

**7.** Verfahren gemäß Anspruch 6, **dadurch gekennzeichnet, dass** die Konzentration der Hydroxyl-Gruppen in dem Ammoniakpuffer im Bereich von 0,0001 M bis 5 M ist.

**8.** Verfahren gemäß Anspruch 6, **dadurch gekennzeichnet, dass** die Konzentration der Hydroxyl-Gruppen in dem Ammoniakpuffer im Bereich von 0,03 M bis 0,5 M ist.

**9.** Verfahren gemäß Anspruch 6, **dadurch gekennzeichnet, dass** die Konzentration der Hydroxyl-Gruppen in dem Ammoniakpuffer im Bereich von 0,04 M bis 0,33 M ist.

**10.** Verfahren gemäß einem der vorhergehenden Ansprüche, **dadurch gekennzeichnet, dass** die analysierten Proben in ansteigender Folge der Konzentration an Kokain oder anderen Alkaloiden angeordnet sind.

**11.** Verfahren gemäß einem der vorhergehenden Ansprüche, **dadurch gekennzeichnet, dass** die Proben Bestätigungsanalysen mit Standardtechniken, wie zum Beispiel GC oder GC/MS, unterliegen.

**12.** Verfahren gemäß einem der vorhergehenden Ansprüche, **dadurch gekennzeichnet, dass** jede Haarprobe hergestellt ist aus ungefähr 50 mg bis 300 mg fein geteiltem und/oder pulverisiertem Haar.

**13.** Verfahren gemäß Anspruch 1, **dadurch gekennzeichnet, dass** das flüssige Reagens eine Lösung ist mit einem gelösten Stoff, der ausgewählt ist aus Aluminiumhydroxyd, Bariumhydroxyd, Octahydrat, Benzyltriethylammoniumhydroxyd, Kalziumhydroxyd, Lithiumhydroxyd, Lithiumhydroxyd-Monohydrat, Magnesiumhydroxyd, Kaliumhydroxyd, Kaliumhydroxydantimoniat, Sodiumhydroxyd, Sodiumhydroxydmonohydrat, Strontiumhydroxydoctahydrat, Tetramethylammoniumhydroxyd, Tetrapropylammoniumhydroxyd, Trimethylvinylammoniumhydroxyd, das gelöst ist in einer Lösung, die ausgewählt ist aus Ethanol, Methanol, Wasser, monobasischem Ammoniumphosphat, Ammoniumacetat, Ammoniumbenzoat, Ammoniumbikarbonat, Ammoniumbichromat, Ammoniumbisulfat, Ammoniumbromid, Ammoniumkarbamid, Ammoniumkarbonat, bibasischem Ammoniumzitrat, Ammoniumchromat, Ammoniumjodid, Molybdat, Ammoniummonovanadat, Ammoniumnitrat, Ammoniumoxalatmonohydrat, Ammoniumpersulfat, Ammoniumsulfat, Ammoniumsulfamat, Ammoniumsulfit, Ammoniumsulfid, Ammoniumtartrat, Ammoniumthiocyanat, Ammoniumthioglycolat, Ammoniumthiosulfat, Ammoniumchlorid, monobasisches Sodiumphosphat, bibasisches Sodiumphosphat, monobasisches Kaliumphosphat, bibasisches Kaliumphosphat.

**14.** Diagnosegerät zum Ausführen des Verfahrens gemäß einem der Ansprüche 1 bis 13 mit einem flüssigen Reagens mit konstanter Konzentration von Hydroxylgruppen, die geeignet sind zum Extrahieren von Kokain und anderen Alkaloiden und Umwandeln von Kokain in Benzoylecgonin und einem herkömmlichen Analysegerät für die Bestimmung von Benzoylecgonin im Urin.

## Revendications

**1.** Procédé, de type dépistage, pour la détermination quantitative de cocaïne et autres alcaloïdes qui sont présents dans un échantillon solide qui comprend les étapes de :

a) préparation d'un échantillon solide dans une forme pulvérulente ou finement divisée ;

b) choix d'un réactif liquide fournissant une concentration constante en groupes hydroxyle adéquats en vue d'extraire et de transformer de la cocaïne en benzoylecgonine et en vue d'extraire d'autres substances similaires ;

c) extraction de la cocaïne et autres substances similaires contenues dans l'échantillon et à transformer la cocaïne extraite en benzoylecgonine en maintenant l'échantillon entièrement immergé dans ledit réactif liquide à une température s'étalant de 10°C à 250°C pendant une période de temps s'étalant de quelques secondes à 48 heures ; et

d) analyse du liquide séparé d'avec l'échantillon solide pour déterminer la concentration en benzoylecgonine contenue dans ledit liquide par rapport au seuil limite en utilisant une trousse de dépistage classique pour la détermination de benzoylecgonine dans les urines.

2. Procédé selon la revendication 1, dans lequel ledit échantillon solide est un échantillon de cheveu.

3. Procédé selon les revendications 1 et 2, dans lequel ladite température s'étale de 100°C à 150°C.

4. Procédé selon les revendications 1 et 2, dans lequel ladite période de temps s'étale de 15 minutes à 24 heures.

5. Procédé selon l'une quelconque des revendications précédentes, dans lequel on maintient ladite température à 100°C pendant 1 heure.

6. Procédé selon la revendication 1, dans lequel ledit réactif liquide est un tampon ammoniaque comprenant (NH4)2HPO4 0,2 M avec l'addition de 5 ml de NH4OH à 25 % à chaque litre de celui-ci.

7. Procédé selon la revendication 6, dans lequel la concentration en groupes hydroxyle dans ledit tampon ammoniaque est dans la gamme allant de 0,0001 M à 5 M.

8. Procédé selon la revendication 6, dans lequel la concentration en groupes hydroxyle dans ledit tampon ammoniaque est dans la gamme de 0,03 M à 0,5 M.

9. Procédé selon la revendication 6, dans lequel la concentration en groupes hydroxyle dans ledit tampon ammoniaque est dans la gamme de 0,04 M à 0,33 M.

10. Procédé selon l'une quelconque des revendications précédentes, dans lequel on dispose les échantillons analysés dans un ordre croissant de concentration en cocaïne ou autres alcaloïdes.

11. Procédé selon l'une quelconque des revendications précédentes, dans lequel on soumet les échantillons à des analyses de confirmation avec des techniques classiques telles que CG ou CG/SM.

12. Procédé selon l'une quelconque des revendications précédentes, dans lequel chaque échantillon de cheveu est fait d'environ 50 mg à 300 mg de cheveu en poudre et/ou finement divisé.

13. Procédé selon la revendication 1, dans lequel ledit réactif liquide est une solution comprenant un soluté choisi parmi l'hydroxyde d'aluminium, l'hydroxyde de baryum octahydraté, l'hydroxyde de benzyltriéthylammonium, l'hydroxyde de benzyltriméthylammonium, l'hydroxyde de calcium, l'hydroxyde de lithium, l'hydroxyde de lithium monohydraté, l'hydroxyde de magnésium, l'hydroxyde de potassium, l'hydroxyantimoniate de potassium, l'hydroxyde de sodium, l'hydroxyde de sodium monohydraté, l'hydroxyde de strontium octahydraté, l'hydroxyde de tetraméthylammonium, l'hydroxyde de tétrapropylammonium, l'hydroxyde de triméthylvinylammonium, dissous dans un solvant choisi parmi l'éthanol, le méthanol, l'eau, le phosphate d'ammonium monobasique, l'acétate d'ammonium, le benzoate d'ammonium, le bicarbonate d'ammonium, le bichromate d'ammonium, le bisulfate d'ammonium, le bromure d'ammonium, le carbamate d'ammonium, le carbonate d'ammonium, le citrate d'ammonium bibasique, le chromate d'ammonium, l'iodure d'ammonium, le molibdate, le monovanadate d'ammonium, le nitrate d'ammonium, l'oxalate d'ammonium monohydraté, le persulfate d'ammonium, le sulfate d'ammonium, le sulfamate d'ammonium, le sulfite d'ammonium, le sulfure d'ammonium, le tartrate d'ammonium, le thiocyanate d'ammonium, le thioglycolate d'ammonium, le thiosulfate d'ammonium, le chlorure d'ammonium, le phosphate de sodium monobasique, le phosphate de sodium bibasique, le phosphate de potassium monobasique, le phosphate de potassium bibasique.

14. Trousse de diagnostic pour la réalisation du procédé selon l'une quelconque des revendications 1 à 13, comprenant un réactif liquide avec une concentration constante en groupes hydroxyle adéquats en vue d'extraire de la cocaïne et autres alcaloïdes et de transformer la cocaïne en benzoylecgonine, et une trousse de dépistage classique pour la détermination de benzoylecgonine dans des échantillons d'urine.

*Fig. 1*

EP 1 196 784 B1

Fig. 2

Fig. 3

Fig. 4

*Fig. 5*

EP 1 196 784 B1

*Fig. 6*

Fig. 7

*Fig. 8*

EP 1 196 784 B1

*Fig. 9*

EP 1 196 784 B1

Fig. 10

*Fig. 11*

*Fig. 12*

*Fig. 13*

EP 1 196 784 B1

COCAINE

INTERMEDIATE

$$CH_3$$ COOCH3

$$N$$ ... COOCH3

COOC6H5

H

OH—

T°C

$$CH_3$$

$$N$$ ... C OCH3

O—

OH

COOC6H5

H

BENZOYLECGONINE

$$CH_3$$

$$N$$ ... COOH

+ CH3OH

COOC6H5

*Fig. 14*

EP 1 196 784 B1

Fig. 15

Fig. 16